# FASCICULE DE BREVET EUROPEEN

(11) **EP 2 713 716 B1**
(45) Date de publication et mention de la délivrance du brevet: **24.08.2016**
(21) Numéro de dépôt: 12731088.6
(22) Date de dépôt: 30.05.2012
(51) Int. Cl.: A01N 1/02

(54) **COMPOSITION COMPRENANT UNE HEMOGLOBINE EXTRACELLULAIRE D'ARENICOLIDAE POUR LA PRESERVATION DES ORGANES ET SON UTILISATION**
ORGANERHALTENDE ZUSAMMENSETZUNG ENTHALTEND EIN EXTRAZELLULÄRES HÄMOGLOBIN VON ARENICOLIDAE UND VERWENDUNGEN DAVON
ORGAN-PRESERVING COMPOSITION COMPRISING AN EXTRACELLULAR HEMOGLOBIN FROM ARENICOLIDAE AND USES THEREFOF

(30) Priorité: 31.05.2011 FR 1154778
(43) Date de publication de la demande: 09.04.2014
(73) Titulaire: Hemarina, 29600 Morlaix (FR)
(72) Inventeur: DUTHEIL, Delphine, F-86800 Saint Julien l'Ars (FR); ROUSSELOT, Morgane, F-29250 Saint Pol de Léon (FR); HAUET, Thierry, F-86550 Miganloux Beauvoir (FR); ZAL, Franck, F-29600 Ploujean-Morlaix (FR)
(74) Mandataire: Cabinet Plasseraud
(86) Numéro de dépôt international: PCT/FR2012/051206
(87) Numéro de publication internationale: WO 2013/001196

(56) Documents cités:
- WO-A1-2007/111495
- WO-A1-2010/128159
- FR-A1- 2 919 785
- ROUSSELOT MORGANE ET AL: "Arenicola marina extracellular hemoglobin: a new promising blood substitute", BIOTECHNOLOGY JOURNAL, WILEY-VCH VERLAG, WEINHEIM, DE, vol. 1, no. 3, 1 janvier 2006 (2006-01-01) , pages 333-345, XP002480915, ISSN: 1860-6768, DOI: 10.1002/BIOT.200500049
- SCHEULE A M ET AL: "Emergency donor heart protection: application of the port access catheter technique using a pig heart transplantation model", TRANSPLANTATION, WILLIAMS AND WILKINS, BALTIMORE US, vol. 77, no. 8, 1 janvier 2004 (2004-01-01), pages 1166-1171, XP002411400, ISSN: 0041-1337, DOI: 10.1097/01.TP.0000122229.79612.DE
- Tariq Hafez, Barry Fuller: "Chapter 9 - Organ Preservation for Transplantation" In: John G. Baust, John M. Baust: "Advances in Biopreservation", 2006, CRC Press, XP009156077, ISBN: 978-0-8493-2772-8 pages 197-270, le document en entier

## Description

La présente invention concerne l'utilisation d'une composition comprenant au moins une globine, un protomère de globine ou une hémoglobine extracellulaire d*'Arenicolidae,* une solution de stabilisation et/ou une solution de conservation d'organes utilisée en clinique, ladite composition ayant une température comprise entre 0°C et 37°C, pour préserver au moins un organe *in situ* chez un donneur décédé en état de mort cérébrale ou décédé d'arrêt cardiaque.

Le don d'organes est le prélèvement d'organes d'un corps humain, appelé donneur, dans le but de traiter un patient, appelé receveur, dont les organes sont gravement atteints.

L'une des difficultés de ce don reste le temps de conservation des organes. En effet, en normothermie (37°C), avant et/ou après prélèvement sur le donneur, un organe subit une période d'ischémie chaude, *i.e.* une période où l'organe n'est plus perfusé par le sang du donneur, et n'est pas encore réfrigéré. Il se détériore rapidement et n'est plus approvisionné en oxygène. Le temps acceptable pour assurer la reprise de fonction ultérieure du greffon varie d'un organe à l'autre, lorsque celui-ci est conservé en hypothermie (i.e. aux alentours de 4°C). Par exemple, il est d'environ 4 à 6 heures pour un coeur ou un poumon, 8 à 12 heures pour un foie, 24 à 48 heures pour un rein et 8 à 10 heures pour un pancréas ou un intestin. La transplantation doit donc s'effectuer dans une durée bien définie, afin d'assurer le maintien de la fonctionnalité de l'organe.

Par ailleurs, l'hypothermie est l'élément essentiel de la conservation. Dès l'explantation, le greffon est refroidi, afin de faire descendre rapidement sa température de 37°C à 4°C ; pour cela, l'organe est rincé avec une solution de préservation via les vaisseaux puis simplement immergé dans cette solution de préservation maintenue à basse température par de la glace pilée selon des conditions garanties d'asepsie. La diminution de la température des tissus entraîne une diminution du métabolisme cellulaire, c'est-à-dire un ralentissement de l'activité enzymatique catalytique nécessaire à la viabilité cellulaire, sans pour autant l'arrêter (Belzer F.O., Southard J.H. Principles of solid-organ préservation by cold storage. Transplantation 1988; 45(4): 673-676.). Le greffon placé à 4°C a une diminution de son métabolisme d'environ 85%. L'hypothermie permet ainsi de lutter contre les effets délétères de la privation en oxygène et en nutriments induits par l'arrêt de la circulation sanguine et diffère la mort des cellules, responsable de la nécrose des tissus.

Face aux pénuries de dons, la préservation du greffon et son oxygénation, sur une période plus longue, sont des préoccupations essentielles ; cela permet un maintien de la qualité de l'organe, sa survie prolongée, et ainsi la réussite de la greffe. En effet, même si le métabolisme d'un greffon conservé à 4°C est réduit, celui-ci a toujours besoin d'oxygène, comme tous les tissus aérobies.

En outre, la majorité des substituts sanguins disponibles aujourd'hui, tels que les perfluorocarbones (PFC), les HBOC ou le sang humain, sont capables d'oxygéner les organes, mais ne sont pas utilisables à n'importe quelle température. Notamment, ils ne sont pas fonctionnels ou stables à 4°C. Par ailleurs, les PFC ne sont pas des transporteurs d'oxygène, mais des solutés capables de dissoudre une quantité importante d'oxygène en fonction de la pression partielle en oxygène. Ils ne peuvent donc pas être mis en oeuvre d'une façon simple, et peuvent créer des problèmes de stress oxydant.

FR2919785 décrit l'utilisation d'une composition comprenant au moins une globine, un protomère de globine ou une hémoglobine extracellulaire d'Annélides et un milieu de conservation d'organes pour la préservation d'un organe lors de transplantations. Cette globine, protomère de globine ou hémoglobine extracellulaire appartient notamment à des vers marines tels que *Arenicola marina.*

WO2010/128159 décrit l'utilisation d'une composition comprenant une hémoglobine extracellulaire de *Nereis virens.* Cette composition comprend aussi une solution de conservation d'organes, comme la solution UW ou la Custodiol. La composition protège des organes contre la détérioration par l'ischémie et/ou anoxie. Elle peut être utilisée pour préserver un organe isolé ou chez un donneur décédé en état de mort cérébrale.

De manière surprenante, les inventeurs ont maintenant découvert que l'administration, de préférence par injection intra-corporelle, d'une composition spécifique, ladite composition ayant une température comprise entre 0°C et 37°C, chez un donneur décédé en état de mort cérébrale ou décédé d'arrêt cardiaque, permet de préserver et d'oxygéner ses organes dans des conditions optimales, afin de conserver leurs fonctions avant leurs collectes chez ledit donneur. La composition spécifique comprend une hémoglobine extracellulaire d*'Arenicolidae,* une solution de stabilisation et/ou une solution de conservation d'organes. Son administration *in situ* permet de conserver les fonctions des organes dans des conditions optimales avant leur prélèvement, et de refroidir lesdits organes ou de les maintenir à toute température comprise entre 0 et 37°C, de préférence en normothermie. La composition selon l'invention, qui contient un transporteur d'oxygène, permet également d'oxygéner de manière efficace les organes *in situ* chez le donneur et d'assurer leur qualité. Enfin, la composition selon l'invention est stable et fonctionnelle à 4°C aussi bien qu'à 37°C.

La présente invention a donc pour objet l'utilisation d'une composition comprenant au moins une globine, un protomère de globine ou une hémoglobine extracellulaire d'*Arenicolidae,* une solution de stabilisation et/ou une solution de conservation d'organes, ladite composition ayant une température comprise entre 0°C et 37°C, pour préserver un organe chez un donneur décédé en état de mort cérébrale ou décédé d'arrêt cardiaque. La préservation de l'organe se fait donc directement *in situ* chez le donneur décédé. La composition selon l'invention peut être directement perfusée chez le donneur en attente de prélèvement des différents greffons (coeur, poumon, foie, reins, pancréas, intestin, cornée...).

L'invention a également pour objet l'utilisation d'une composition comprenant au moins une globine, un protomère de globine ou une hémoglobine extracellulaire d'*Arenicolidae,* une solution de stabilisation et/ou une solution de conservation d'organes, ladite composition ayant une température comprise entre 0°C et 37°C, pour préparer une composition pharmaceutique pour préserver au moins un organe chez un donneur décédé en état de mort cérébrale ou décédé d'arrêt cardiaque. La présente invention a également pour objet une composition comprenant au moins une globine, un protomère de globine ou une hémoglobine extracellulaire d'*Arenicolidae,* une solution de stabilisation et/ou une solution de conservation d'organes, ladite composition ayant une température comprise entre 0°C et 37°C, pour son utilisation pour préserver au moins un organe chez un donneur décédé en état de mort cérébrale ou décédé d'arrêt cardiaque.

Est également décrite l'utilisation d'une solution aqueuse comprenant du chlorure de sodium, du chlorure de calcium, du chlorure de magnésium, du chlorure de potassium, du sodium gluconate et du sodium acétate, et éventuellement un ou des antioxydants, ladite solution aqueuse ayant un pH compris entre 6.5 et 7.6, de préférence égal à 7,1 ± 0,5, de préférence d'environ 7.35, pour préserver au moins un organe *in situ* chez un donneur décédé en état de mort cérébrale ou décédé d'arrêt cardiaque. Cette solution aqueuse peut être combinée à une solution de conservation d'organes, i.e. elle peut être mélangée à une solution de conservation d'organes. Préférentiellement, ladite solution aqueuse comprend 90 mM de NaCl, 23 Mm de Na-gluconate, 2,5 mM de CaCl₂, 27 mM de Na-acétate, 1,5 mM de MgCl₂, 5 mM de KCI, et a un pH de 7,1 ± 0,5, et éventuellement entre 0 et 100 mM d'antioxydant de type acide ascorbique et/ou gluthation réduit. Ladite solution a de préférence une osmolarité comprise entre 300 et 350, et préférentiellement de 302 mOsmol/L.

Selon l'invention, la préservation de l'organe s'effectue directement chez le donneur post mortem. Le donneur décédé peut être en état de mort cérébrale, ou en arrêt cardiaque. Dans ce dernier cas, on parle de prélèvement d'organes à coeur arrêté.

La mort cérébrale, appelée également coma dépassé ou coma de type IV, est définie comme l'état de cessation complète et définitive, irréversible, de l'activité cérébrale, alors que la circulation sanguine persiste. Un donneur est en état de mort cérébrale, ou mort encéphalique, lorsque l'encéphale est détruit de manière irréversible, malgré la persistance temporaire d'une activité hémo-dynamique et d'une vascularisation des organes.

Un donneur est en arrêt cardiaque si cet arrêt cardiaque est irréversible après arrêt des mesures de réanimation. Le délai après lequel une asystolie est considérée comme irréversible est de l'ordre d'une minute, après arrêt des mesures de réanimation. Les recommandations exigent toutefois plus de 5 minutes de délai.

La composition selon l'invention comprend :
- au moins une globine, un protomère de globine ou une hémoglobine extracellulaire d'*Arenicolidae*, et
- une solution de stabilisation et/ou une solution de conservation d'organes.

La composition selon l'invention peut ainsi comprendre soit au moins une globine, un protomère de globine ou une hémoglobine extracellulaire d'*Arenicolidae*, et une solution de stabilisation ; soit au moins une globine, un protomère de globine ou une hémoglobine extracellulaire d*'Arenicolidae,* et une solution de conservation d'organes ; soit au moins une globine, un protomère de globine ou une hémoglobine extracellulaire d'*Arenicolidae*, une solution de stabilisation et une solution de conservation d'organes.

La composition selon l'invention comprend donc au moins un composé choisi parmi l'hémoglobine extracellulaire d'*Arenicolidae,* ses protomères de globine et ses globines.

L'hémoglobine extracellulaire d'Annélides est présente chez les trois classes d'Annélides : les Polychètes, les Oligochètes et les Achètes. On parle d'hémoglobine extracellulaire car elle est naturellement non contenue dans une cellule, et peut donc circuler librement dans le système sanguin sans modification chimique pour la stabiliser ou la rendre fonctionnelle.

L'hémoglobine extracellulaire d'Annélides est un biopolymère géant de poids moléculaire compris entre 2000 et 4000 kDa, constitué d'environ 200 chaînes polypeptidiques comprises entre 4 et 12 types différents que l'on regroupe généralement en deux catégories.

La première catégorie, comptant 144 à 192 éléments, regroupe les chaînes polypeptidiques dites "fonctionnelles" qui portent un site actif de type hème, et sont capables de lier réversiblement l'oxygène ; ce sont des chaînes de type globine dont les masses sont comprises entre 15 et 18 kDa et qui sont très similaires aux chaînes de type α et β de vertébrés.

La deuxième catégorie, comptant 36 à 42 éléments, regroupe les chaînes polypeptidiques dites de "structure" ou « linkers » possédant peu ou pas de site actif mais permettant l'assemblage des sous-unités appelées douzièmes ou protomères.

Chaque molécule d'hémoglobine est constituée de deux hexagones superposés que l'on a nommés bicouche hexagonale (hexagonal-bilayer) et chaque hexagone est lui-même formé par l'assemblage de six sous-unités (ou "douzièmes" ou « protomères ») en forme de goutte d'eau. La molécule native est formée de douze de ces sous-unités (dodécamère ou protomère). Chaque sous-unité a une masse moléculaire comprise entre 200 et 250 kDa, et constitue l'unité fonctionnelle de la molécule native.

Selon l'invention l'hémoglobine extracellulaire d'Annélides est choisie parmi les hémoglobines extracellulaires de la famille des *Arenicolidae.* Encore plus préférentiellement, l'hémoglobine extracellulaire d'Annélides est l'hémoglobine extracellulaire d'*Arenicola marina.*

Selon l'invention, la composition peut également comprendre au moins un protomère de globine de l'hémoglobine extracellulaire *d'Arenicolidae.* Ledit protomère constitue l'unité fonctionnelle de l'hémoglobine native, comme indiqué ci-dessus.

Enfin, la composition peut également comprendre au moins une chaîne de globine de l'hémoglobine extracellulaire *d'Arenicolidae.* Une telle chaîne de globine peut notamment être choisie parmi les chaînes de globine de type Ax et/ou Bx d'hémoglobine extracellulaire d'*Arenicolidae*.

L'hémoglobine extracellulaire d'Annélides et ses protomères de globine ont une activité superoxide dismutase (SOD) intrinsèque, et ne nécessitent, par conséquent, aucun antioxydant pour fonctionner, contrairement à l'utilisation d'une hémoglobine de mammifère, pour laquelle les molécules antioxydantes sont contenues à l'intérieur du globule rouge et ne sont pas liées à l'hémoglobine. D'autre part, l'hémoglobine extracellulaire d'Annélides, ses protomères de globine et/ou ses globines ne nécessitent pas de cofacteur pour fonctionner, contrairement à l'hémoglobine de mammifère, notamment humaine. Enfin, l'hémoglobine extracellulaire d'Annélides, ses protomères de globine et/ou ses globines ne possédant pas de typage sanguin, ils permettent d'éviter tout problème de réaction immunologique.

La composition selon l'invention peut également comprendre une solution de stabilisation, dont la composition est compatible avec une solution de conservation d'organes. Cette solution de stabilisation crée un environnement salin adéquat pour l'hémoglobine, ses protomères et ses globines, et permet ainsi le maintien de la structure quaternaire, et donc de la fonctionnalité de cette molécule. Grâce à la solution de stabilisation, l'hémoglobine, ses protomères et ses globines sont capables d'assurer leur fonction d'oxygénation des organes.

La solution de stabilisation selon l'invention est une solution aqueuse comprenant des sels, de préférence des ions chlorure, sodium, calcium, magnésium et potassium, et confère à la composition selon l'invention un pH compris entre 6.5 et 7.6 ; sa formulation est similaire à celle d'un liquide physiologiquement injectable, et elle peut être utilisée seule comme solution de conservation, ou en combinaison avec l'hémoglobine, ou en combinaison avec une solution de préservation d'organes commerciale. Dans ces conditions, l'hémoglobine extracellulaire *d'Arenicolidae,* ses protomères de globine et ses globines restent fonctionnels, et la composition selon l'invention, administrée entre 0 °C et 37°C au donneur, est compatible avec les organes à préserver et à oxygéner.

Dans la présente description, le pH s'entend à température ambiante (25°C), sauf mention contraire.

De préférence, la solution de stabilisation est une solution aqueuse comprenant du chlorure de sodium, du chlorure de calcium, du chlorure de magnésium, du chlorure de potassium, ainsi que du sodium gluconate et du sodium acétate, et a un pH compris entre 6.5 et 7.6, de préférence égal à 7,1 ± 0,5, de préférence d'environ 7.35. Plus préférentiellement, la solution de stabilisation est une solution aqueuse comprenant 90 mM de NaCl, 23 Mm de Na-gluconate, 2,5 mM de CaCl₂, 27 mM de Na-acétate, 1,5 mM de MgCl₂, 5 mM de KCI, et a un pH de 7,1 ± 0,5, pouvant contenir entre 0 et 100 mM d'antioxydant de type acide ascorbique et/ou gluthation réduit. Ladite solution a de préférence une osmolarité comprise entre 300 et 350, et préférentiellement de 302 mOsmol/L.

La composition selon l'invention peut enfin comprendre une solution de conservation d'organes. Cette solution permet de maintenir le métabolisme de base des cellules constitutives du greffon. Elle répond à un triple objectif : assurer le lavage du sang artériel du greffon, amener de façon homogène le greffon à la température de conservation désirée, et protéger et prévenir des lésions provoquées par l'ischémie et la reperfusion et optimiser la reprise de fonction. La solution de conservation d'organes est donc cliniquement acceptable.

La solution de conservation d'organes est une solution aqueuse ayant un pH compris entre 6.5 et 7.5, comprenant des sels, de préférence des ions chlorure, sulfate, sodium, calcium, magnésium et potassium ; des sucres, de préférence le mannitol, le raffinose, le saccharose, le glucose, le fructose, le lactobionate (qui est un imperméant), ou le gluconate ; des antioxydants, de préférence le glutathion ; des agents actifs, de préférence des inhibiteurs de xanthine oxydase tel que l'allopurinol, des lactates, des acides aminés tel que l'histidine, l'acide glutamique (ou glutamate), le tryptophane ; et éventuellement des colloïdes tels que de l'hydroxyéthyl amidon, du polyéthylène glycol ou du dextran.

Selon un mode de réalisation préféré de l'invention, la solution de conservation d'organes est choisie parmi :
- la solution de l'Université du Wisconsin (UW ou Viaspan^{®}), qui a une osmolalité de 320 mOsmol/kg et un pH de 7.4, de formulation suivante pour un litre, dans l'eau :

| |
|---|
| Lactobionate de potassium: 100 mM |
| KOH: 100 mM |
| NaOH: 27 mM |
| KH₂PO₄: 25 mM |
| MgSO₄: 5mM |
| Raffinose: 30 mM |
| Adénosine: 5 mM |
| Glutathion: 3 mM |
| Allopurinol: 1 mM |
| Hydroxyéthyl amidon: 50 g/L, |

- IGL-1^{®}, ayant une osmolalité de 320 mOsm/kg et un pH de 7.4, de formulation suivante, pour un litre dans l'eau :

| |
|---|
| NaCl : 125 mM |
| KH₂PO₄: 25 mM |
| MgSO₄: 5 mM |
| Raffinose: 30 mM |
| Lactobionate de potassium: 100 mM |
| Glutathion: 3 mM |
| Allopurinol: 1 mM |
| Adénosine: 5 mM |
| Polyéthylène glycol (poids moléculaire: 35 kDa): 1 g/L, |

- Celsior^{®}, ayant une osmolalité de 320 mOsm/kg et un pH de 7.3, de formulation suivante pour un litre dans l'eau :

| |
|---|
| Glutathion: 3 mM |
| Mannitol : 60 mM |
| Acide lactobionique: 80 mM |
| Acide glutamique : 20 mM |
| NaOH: 100 mM |
| Chlorure de calcium dihydraté: 0.25 mM |
| MgSO₄: 1.2 mM |
| KCl: 15 mM |
| Chlorure de magnésium hexahydraté: 13 mM |
| Histidine : 30 mM, |

- SCOT 15 Multi Organes Abdominaux^{®} et SCOT 30 Greffons Vasculaires^{®} de Macopharma, comprenant notamment tous deux du polyéthylène glycol de haut poids moléculaire (20 kDa),
- BMPS Belzer^{®}, ou Belzer machine perfusion solution, ou KPS1, comprenant notamment 100 mEq/L de sodium, 25 mEq/L de potassium, un pH de 7.4 à température ambiante, et ayant une osmolarité de 300 mOsm/L,
- Custodiol^{®} HTK Solution, de formulation suivante pour un litre dans l'eau, le pH étant de 7.20 à température ambiante, et l'osmolalité étant de 310 mOsm/kg:

| |
|---|
| NaCl : 18.0 mM |
| KCl : 15.0 mM |
| KH₂PO₄ : 9 mM |
| 2-cétoglutarate de potassium hydrogéné : 1.0 mM |
| Chlorure de magnésium hexahydraté: 4.0 mM |
| Histidine,HCl,H2O : 18.0 mM |
| Histidine: 198.0 mM |
| Tryptophane: 2.0 mM |
| Mannitol: 30.0 mM |
| Chlorure de calcium dihydraté: 0.015 mM, |

- Euro-Collins^{®}, ayant une osmolalité de 355 mOsm/kg et un pH de 7.0, et de formulation suivantepour un litre dans l'eau:

| |
|---|
| Sodium : 10 mM |
| Potassium: 115 mM |
| Chlorure: 15 mM |
| H₂PO₄⁻: 15 mM |
| HPO₄²⁻ : 42.5 mM |
| HCO₃⁻ : 10 mM |
| Glucose: 194 mM, |

- Soltran^{®}, ayant une osmolalité de 486 mOsm/kg et un pH de 7.1, et de formulation suivante pour un litre dans l'eau:
Sodium : 84 mM
Potassium: 80 mM
Magnésium: 41 mM
Sulfate⁻ : 41 mM
Mannitol: 33,8 g/l
Citrate : 54 mM
Glucose: 194 mM,

- Perfadex^{®}, ayant une osmolarité de 295 mOsmol/L et de formulation suivante dans l'eau :
50g/L de dextran 40 (poids moléculaire : 40.000),
Na+ : 138 mM,
K+ : 6 mM,
Mg2+ : 0.8 mM,
Cl- : 142 mM,
SO₄²⁻ : 0.8 mM,
(H₂PO₄⁻ + HPO₄²⁻) : 0.8 mM et
Glucose : 5 mM,

- Ringer lactate^{®}, de formulation suivante, dans l'eau, le pH étant compris entre 6.0 et 7.5 à température ambiante, et ayant une osmolarité de 276.8 mOsmol/L :
Na+ : 130 mM,
K+ : 5.4 mM,
Ca2+ : 1.8 mM,
CI- : 111 mM,
Lactates : 27.7 mM,

- Plegisol^{®}, de formulation suivante, dans l'eau :
KCl : 1.193 g/L,
MgCl₂, 6 H₂O : 3.253 g/L,
NaCl: 6.43 g/L,
CaCl₂: 0.176 g/L,

- Solution de l'Hôpital Edouard Henriot, de formulation suivante dans l'eau, le pH étant égal à 7.4 à température ambiante, et ayant une osmolarité de 320 mOsmol/L :
KOH : 25mM,
NaOH : 125mM,
KH₂PO₄ : 25mM,
MgCl₂ : 5mM,
MgSO₄ : 5mM,
Raffinose : 30mM,
Lactobionate: 100mM,
Glutathion: 3mM,
Allopurinol: 1mM,
Adenosine: 5mM,
Hydroxyéthyl amidon 50g/L,

- et la solution Steen®, comprenant de la sérum albumine humaine, du dextran et des électrolytes extracellulaires avec une faible concentration de potassium.

Toutes ces solutions de conservation d'organes sont des produits commerciaux.

De préférence, la composition selon l'invention comprend (i) la solution de stabilisation et (ii) la solution de conservation d'organes, de préférence l'une des solutions commerciales décrites ci-dessus, dans un ratio pondéral (i) : (ii) compris entre 0 : 100 et 100 : 0.

De préférence, la composition selon l'invention a un pH compris entre 6,5 et 7,6, et comprend :
- au moins une globine, un protomère de globine ou une hémoglobine extracellulaire d'*Arenicolidae,*
- des ions calcium, de préférence en quantité comprise entre 0 et 0.5 mM ;
- du KOH, de préférence en quantité comprise entre 20 et 100 mM ;
- du NaOH, de préférence en quantité comprise entre 20 et 125 mM ;
- du KH₂PO₄, de préférence en quantité comprise entre 20 et 25 mM ;
- du MgCl2, de préférence en quantité comprise entre 3 et 5 mM ;
- au moins un sucre choisi parmi le raffinose et le glucose, de préférence en quantité comprise entre 5 et 200 mM ;
- de l'adénosine, de préférence en quantité comprise entre 3 et 5 mM ;
- du glutathion, de préférence en quantité comprise entre 2 et 4 mM ;
- de l'allopurinol, de préférence en quantité comprise entre 0 et 1 mM ; et
- au moins un composé choisi parmi l'hydroxyéthyl amidon, les polyéthylène glycols de différents poids moléculaires et la sérum albumine humaine, de préférence en quantité comprise entre 1 et 50 g/L.

Typiquement, l'hémoglobine extracellulaire d'*Arenicolidae,* ses protomères de globine et/ou ses globines, est présente à une concentration, par rapport au volume final de composition, comprise entre 0,001 mg/ml et 100 mg/ml, préférentiellement entre 0,005 mg/ml et 20 mg/ml, plus préférentiellement entre 1 mg/ml et 5 mg/ml, en particulier 1 mg/ml.

Typiquement, la composition selon l'invention a une osmolarité comprise entre 250 et 350 mOsm/L, de préférence comprise entre 275 et 310 mOsm/L, de préférence d'environ 302 mOsm/L.

De préférence, la composition selon l'invention comprend (i) l'hémoglobine extracellulaire d'*Arenicolidae,* ses protomères de globine et ses globines, (ii) la solution de conservation d'organes et (iii) la solution de stabilisation, pour une dose d'hémoglobine comprise entre 0 g/L et 150g/L, pour une dilution de la solution de conservation d'organes dans un ratio volumétrique de 8x10⁻³ à 100 pour cent.

Selon un mode de réalisation préféré, la température des compositions selon l'invention est comprise entre 0°C et 37°C, préférentiellement entre 2°C et 32°C, préférentiellement entre 4°C et 25°C, et plus préférentiellement environ 4°C.

Les compositions selon l'invention permettent de travailler aussi bien en condition hypothermique, que normothermique (proche de la température physiologique).

L'invention a également pour objet un procédé de conservation d'un organe *ex situ* dans un donneur décédé en état de mort cérébrale ou décédé d'arrêt cardiaque, comprenant les étapes suivantes:
a) perfusion dudit donneur décédé avec une composition comprenant au moins une globine, un protomère de globine ou une hémoglobine extracellulaire d'*Arenicolidae,* une solution de stabilisation et/ou une solution de conservation d'organes, ladite composition ayant une température comprise entre 0°C et 37°C, préférentiellement entre 2°C et 5°C, plus préférentiellement d'environ 4°C,
   ou avec une solution aqueuse comprenant du chlorure de sodium, du chlorure de calcium, du chlorure de magnésium, du chlorure de potassium, du sodium gluconate et du sodium acétate, et éventuellement un ou des antioxydants, ladite solution aqueuse ayant un pH compris entre 6.5 et 7.6, de préférence égal à 7,1 ± 0,5, de préférence d'environ 7.35; puis
b) prélèvement de l'organe à transplanter ; puis
c) conservation en statique ou en perfusion dynamique dudit organe obtenu en b), à une température comprise entre 0°C et 37°C, préférentiellement comprise entre 2°C et 25°C, plus préférentiellement d'environ 4°C, pendant un temps déterminé en fonction dudit organe, dans la composition ou la solution aqueuse définie à l'étape a).

Par « temps déterminé en fonction dudit organe », on entend un temps de conservation qui est spécifique et dépend de l'organe à transplanter, comme indiqué ci-avant.

La composition selon l'invention est de préférence administrée par injection, notamment par injection intra-vasculaire. Elle peut être administrée également par circulation régionale normothermique (CRN), i.e. avec un cathéter artériel, un cathéter veineux et un cathéter de Fogarty, ou par circulation régionale hypothermique (CRH) pour un refroidissement généralisé du donneur. Elle peut également être administrée à l'aide d'une sonde de Gillot ou toute autre technique similaire.

Le système de CRN ou CRH est une technique de circulation extracorporelle du fluide sanguin.

La sonde de Gillot permet quant à elle un refroidissement *in situ* des organes abdominaux. Notamment, lorsqu'elle est implantée dans le circuit artériel fémoral, elle permet d'isoler la circulation rénale. À travers la sonde de Gillot, une perfusion rapide de la composition selon l'invention peut être réalisée. Une voie de décharge est mise en place au niveau de la veine fémorale permettant l'évacuation de la composition liquide perfusée.

L'invention est maintenant illustrée à l'aide des exemples ci-dessous.

### Exemple 1 :

### Matériel & Méthodes

### Production et utilisation de M101 dans des solutions de conservation

M101 (HEMO2life ®, Hemarina SA, France) est une solution tamponnée d'hémoglobine *d'Arenicola marina,* et a été fabriquée en utilisant des procédures standard pour l'extraction des produits biologiques conformes aux spécifications des autorités sanitaires. La protéine purifiée est congelée à -80°C puis décongelée à 4°C avant l'expérience et diluée dans une solution de conservation: UW (ViaSpan®, Bristol Myers Squibb, Belgique), HTK (Custodiol®, Allemagne), IGL (IGL-1®, Institut Georges Lopez, France), Celsior (Celsior®, Genzyme, France), Ringer Lactate (RL, Aguettant, France) ou Perfadex (Perfadex®, Vitrolife, Suède).

### Analyses fonctionnelles de M101

### Fixation de l'oxygène

M101 a été ajoutée (1g/L) à la solution UW. Le gaz N2 a été utilisé pour désoxygéner la solution, ou bien des cellules LLC-PK1 ont été incorporées à la préparation, puis les deux préparations ont été hermétiquement fermées. La fonctionnalité de M101 a été suivie par spectrophotométrie, [35] permettant la caractérisation de l'oxyhémoglobine et de la désoxyhémoglobine. Les spectres d'absorption ont été enregistrés sur la gamme 390-650nm (UVmc2, SAFAS, Monaco). L'O2 dissous (dO2) a été contrôlé à l'aide d'un capteur d'O2 (Metler Toledo, France).

### Activité de la SOD

L'activité SOD de M101 a été évaluée par un dosage au tétrazolium nitro bleu (NBT) modifié par Oberley et Spitz [36]. En bref, du superoxyde a été généré par la xanthine et la xanthine oxydase en présence de la catalase et DETAPAC. La réduction du NBT a été détectée par spectrophotométrie à 560nm. Du KCN a été ajouté pendant 1h à 4°C avant de commencer l'expérience pour désactiver le complexe Cu/Zn-SOD. Un complexe Cu/Zn-SOD issu d'érythrocytes de boeuf a été utilisé comme contrôle (Calbiochem). une diminution de l'absorbance indique une augmentation de l'activité de piégeage. Le pourcentage d'inhibition de la production d'anion superoxyde a été calculé à l'aide de la formule: [(A0-A1) / A0x100], où A0 est l'absorbance du contrôle et A1 est l'absorbance des échantillons.

### Analyses structurales de M101

M101 a été ajoutée (1g/L) à des solutions, sa structure a été suivie au fil du temps par filtration sur gel isocratique à température ambiante avec un échantillonneur automatique fixé à 4°C utilisant un système HPLC (Dionex, France) et une colonne Superose 6C 1cmx30cm (plage de fractions 5-5000kDa, GE Healthcare, France). Le débit était de 0,5 ml / min et l'éluat a été suivi par un détecteur à photodiodes sur la gamme 250-700nm. Les courbes d'élution ont été acquises et traitées à l'aide du logiciel Chromeleon® (Dionex).

La courbe de dissociation a été obtenue par normalisation de la surface du pic de M101 à l'instant t (At) avec la surface du pic de M101 au temps 0 (At0), et tracée en fonction du temps. Le logiciel Prism GraphPad (logiciels GraphPad, USA) a été utilisé pour ajuster la courbe sur un profil linéaire (f(At/At0) =- kd.t, T ½= 1 / (2.kd)) ou mono-exponentiel (f(At/ At0) = a.exp [-kd.t]). L'acceptabilité a été jugée avec le meilleur coefficient de corrélation. La constante de dissociation (kd) et la demi-vie (T ½) ont été déduites de la meilleure courbe ajustée: linéaire ou mono-exponentielle.

### Expériences sur cellules à 4°C

Des cellules tubulaires proximales porcines LLC-PK1 (CL-101, ATCC, LGC Standards, France) ont été cultivées comme précédemment décrit [37]. Les lésions d'ischémie froide ont été simulées en stockant une monocouche de cellules à 4°C sous atmosphère ambiante dans une solution de préservation (UW, HTK, IGL, Celsior, RL ou Perfadex) additionnée ou non de M101.

Les dosages ont été:
- pour la nécrose: la libération de lactate déshydrogénase (LDH) a été testée en utilisant un Kit de Dosage In Vitro de Toxicologie;
- pour l'apoptose: l'activité caspase-3 a été déterminée en utilisant la trousse de dosage Caspase-3 fluorimétrique (R & D Systems, France);
- pour la viabilité: l'activité métabolique a été déterminée par le test MTT ;
- pour le contenu énergétique : l'ATP intracellulaire a été déterminée en utilisant le Kit test bioluminescent d'adénosine 5'-triphosphate (ATP). Les kits ont été utilisés selon les directives du fabricant. Les réactions ont été quantifiées avec un lecteur multiple (Victor3, Perkin-Elmer, France).

Pour chaque paramètre, les résultats sont exprimés en pourcentage des valeurs mesurées dans les cellules conservées à froid par rapport à la valeur mesurée dans les cellules avant la lésion (contrôle).

### Interventions chirurgicales in vivo et groupes expérimentaux

De porcs mâles de type Large White (INRA/GEPA, Surgères, France) ont été préparés comme précédemment décrit [4], conformément aux recommandations françaises de la Commission d'Ethique pour l'Homme et des Etudes chez l'Animal. Le rein droit a été recueilli, le froid a été injecté et conservé pendant 24 heures avant la transplantation ; ce temps a été choisi parce qu'il est un peu plus long que la durée d'ischémie froide recueillie par United Network for Organ Sharing pour les allogreffes rénales (19,6 ± 8.4h en 2000 [43]). Le rein gauche a été néphrectomisé pour imiter la masse de rein en situation transplantée. Les équipes chirurgicales ont été aveuglées sur les protocoles. Le temps pour les anastomoses vasculaires était de 30 ± 5 minutes, la perte de sang a été minime et aucune complication post-opératoire n'a été observée. Quatre groupes ont été étudiés:
1-UW: conservation des organes avec UW;
2-UW + M101 : UW complété avec 5g/L de M101;
3-HTK: HTK;
4-HTK + M101: HTK complété avec 5g/L de M101.

Les contrôles ont été les animaux opérés de manière fictive.

### Paramètres fonctionnels

Les porcs ont été placés dans une cage métabolique pour les mesures de diurèse (ml/24h), de créatininémie (mol / L), de fraction excrétée du sodium (%) et de protéinurie (g/24h), comme décrit précédemment [3-5].

### Etudes morphologiques

Les biopsies ont été recueillies 7, 14 jours et 1 mois après la reperfusion. La perte des frontières et le détachement endoluminal ont été évalués en utilisant une échelle semi-quantitative de 6 points: 0 - aucune anomalie; 1 - lésions légères affectant moins de 25% des échantillons de reins; 2 - lésions affectant 25-50% des échantillons de reins; 3 - lésions affectant 51-75% des échantillons de reins; 4 - lésions affectant plus de 75% des échantillons de rein et 5 - une nécrose étendue et des lésions rénales [38].

La détermination quantitative de l'invasion interstitielle a été adaptée de la classification de Banff [39]: 0 - Pas de cellules mononucléaires inflammatoires dans les tubules; 1 - Les foyers avec 1 à 4 cellules mononucléées par section transversale tubulaire ou dix cellules tubulaires; 2 - Les foyers avec 5 à 10 cellules mononucléées par section transversale tubulaire et 3 - Les foyers de plus de 10 cellules mononucléées par section tubulaire. La fibrose tubulointerstitiale été déterminée en utilisant la coloration de Picro Sirius [40]. L'immunohistochimie a été utilisée pour la mesure de l'invasion de cellules ED1+ et CD3+ (SouthernBiotech, USA). L'évaluation quantitative a été effectuée sur les champs de haute tension 5-10 (200X).

### Méthodes statistiques

La moyenne ± SEM ou SD est indiquée. Les données *in vitro* ont été comparées en utilisant le test de Dunett. Pour des données *in vivo,* le test de Mann-Whitney U a été utilisé pour des comparaisons entre 2 groupes (uniquement les comparaisons UW par rapport à UW + M101, et HTK versus HTK + M101, ont été effectuées). Des corrélations ont été mesurées avec le test de Spearman et la dépendance de l'effet de M101 sur la solution utilisée a été testée en utilisant un test ANOVA. Les logiciels SPSS (IBM, USA) et Graphpad (Graphpad, USA) ont été utilisés pour les analyses statistiques. La significativité a été acceptée pour p <0,050.

### Résultats

### Fonctionnalités de M101

### Liaison à O2 :

La molécule M101 en solution avec UW est désoxygénée par bullage avec N2 ou par addition de cellules LLC-PK1. Les données obtenues avec les cellules donnent les mêmes résultats qu'avec N2. Dans les conditions atmosphériques ambiantes, M101 dissous dans UW est sous conformation HbO2 (normoxie). Ensuite, la préparation a été fermée hermétiquement et l'O2 consommé par les cellules (hypoxie). Après 75min d'hypoxie, le spectre de M101 passe progressivement vers la conformation désoxy-Hb avec un décalage de la bande de Soret, une diminution des bandes Bêta et Alpha, et une augmentation de l'absorbance autour de 555nm (données non montrées). La désoxygénation complète est apparue après 90min d'hypoxie: le spectre montre un Soret avec un maximum à 428nm et un plateau avec un maximum à 555nm, soit un spectre caractéristique d'un dérivé de désoxy-Hb. Cet état est réversible, car l'oxygénation de la préparation entraîne le retour au spectre initial de M101. La mesure de la dO2 est corrélée aux spectres d'absorption lumineuse (données non présentées).

### Activité de la SOD (données non montrées) :

M101 est un antioxydant efficace, avec une inhibition totale de la formation de NBT (93,5 ± 1,1%). Cette activité est relative à Cu/Zn-SOD car KCN, un inhibiteur spécifique de l'activité enzymatique de Cu/Zn-SOD, inhibe totalement la capacité de piégeage de M101.

### M101 est stable dans les solutions de préservation commerciales (données non montrées)

La stabilité de M101 a été analysée dans UW, HTK, IGL, Celsior, RL et Perfadex. Les constantes de dissociation et demi-vie de M101 montre qu'elle est stable pendant de longues périodes de temps.

### M101 protège les cellules /n vitro contre les lésions dues à la conservation par le froid (données non montrées)

La conservation des cellules épithéliales de rein dans la solution standard, UW, a été très néfaste. L'évaluation de la viabilité cellulaire par libération de LDH a démontré une perte de viabilité des cellules après 12h de CS. Aucune activation significative de la caspase-3 a été détectée. L'activité métabolique et la teneur en ATP ont été réduites de façon concomitante à la libération de LDH.

M101 protège contre ces événements: seulement 0.312g/L ont été suffisants pour améliorer de façon significative l'intégrité structurale et métabolique et le contenu énergétique après 24 heures. La protection totale a été atteinte à 1,25 g/L de M101 (libération de LDH: 6 ± 8%; test MTT: 71 ± 13% et teneur en ATP: 78 ± 23%). Le contenu énergétique cellulaire est augmenté avec des concentrations supérieures à 2,5 g/L (teneur en ATP> 120% par rapport au contrôle). M101 a également protégé les cellules rénales de façon dépendante du temps: l'intégrité cellulaire est totalement préservée (libération de LDH <20%) pendant 24 heures à 1,25 g/L, pendant 36h à 2,5 g/L, pendant 48h à 5 g/L et pendant 72 heures à 10 g/L.

Des expériences ont été reproduites avec d'autres solutions: RL, Perfadex, HTK, IGL et Celsior. Comme pour UW, la conservation dans ces solutions induit des dommages cellulaires structuraux et/ou fonctionnels. Deux types de résultats ont été obtenus: 1) de la même façon que pour UW, les cellules stockées à froid dans RL et, dans une moindre mesure, dans Perfadex, présentent à la fois des dommages structuraux et fonctionnels; 2) des cellules stockées à froid dans HTK, IGL ou Celsior présentent seulement des lésions fonctionnelles. Dans chaque solution, la supplémentation en M101 protège à la fois l'intégrité des cellules et la fonctionnalité des cellules (libération de LDH <20% dans toutes les conditions et test MTT: 50-100%).

### Récupération de la fonction rénale du greffon in vivo plus rapide avec M101

Les porcs transplantés avec un rein préservé avec le mélange UW + M101 reprennent une production d'urine au jour 1 (contre le jour 2 pour UW), et ont eu une récupération plus rapide jusqu'à des niveaux stables de production d'urine au jour 4 (données non montrées, p = 0,016). Les groupes HTK ont eu un recouvrement de diurèse équivalent. Les taux sériques de créatinine dans le groupe UW ont montré des niveaux élevés atteignant un sommet au jour 3, et une diminution lente par la suite. Les animaux UW + M101 ont montré des niveaux significativement plus bas atteignant leur sommet au jour 1, et ont récupéré les niveaux pré-greffe au jour 7 (p = 0,009 à tous les temps, données non montrées). Les animaux HTK ont montré un pic sérique élevé de créatinine au jour 1, suivie d'une lente récupération au-dessus des niveaux pré-greffe, alors que les animaux HTK + M101 ont un pic nettement inférieur au jour 1 (p = 0,009), et une récupération plus rapide jusqu'au niveau pré-greffe au jour 11. Les mesures de réabsorption du sodium ont montré une performance nettement supérieure chez les reins conservés avec M101 par rapport à la solution seule.

### L'intégrité des tissus est mieux préservée chez les greffes avec M101

L'évaluation de la perte de la bordure en brosse et du détachement des cellules, i.e. lésions typiques tubulaires IRI, a révélé des dommages importants dans les greffes UW aux jours 7 et 14, pour se stabiliser au mois 1. Les reins conservés dans UW + M101 présentaient des lésions moins étendues. Les groupes HTK ont montré une tendance similaire à l'amélioration des lésions histologiques par M101.

### L'inflammation est moins sévère dans les reins avec M101

Il y a eu un développement important de la réponse immunitaire dans les greffes UW pendant toute la durée du suivi. Les reins conservés dans UW + M101 ont montré peu d'infiltration immunitaire dès le début, et des signes réduits d'inflammation par la suite. Les deux groupes HTK ont montré un faible niveau d'infiltration. A 3 mois, l'invasion des cellules immunitaires innées (ED1+) et adaptatives (CD3+) révèle une diminution des taux d'invasion dans les reins conservés avec M101 par rapport à la solution seule.

### Résultat amélioré par la supplémentation en M101

Les porcs ont été sacrifiés à 3 mois, i.e. au moment où les inventeurs ont montré le développement d'une fibrose chronique dans ce modèle [41, 42]. Le développement de la fibrose interstitielle et de l'atrophie tubulaire (IFTA) dans les greffons UW a été étendu (23%), tandis que la supplémentation en M101 l'a réduit de manière significative (11%, p = 0,049). Les reins HTK ont également montré un important développement IFTA (25%). Ici aussi, l'ajout de M101 a réduit significativement le développement des dommages (10%, p = 0,038).

Les dommages histologiques ont été associés à une perte chronique de la fonction, puisque les deux groupes HTK et UW ont montré des niveaux élevés de créatinine sérique et de protéinurie. Pour les deux solutions, la supplémentation en M101 a réduit significativement ces niveaux.

### La supplémentation en M101 est en corrélation avec de meilleurs résultats au début et après 3 mois

D'autres analyses statistiques ont montré que la supplémentation en M101 était corrélée négativement avec les taux de créatinine au jour 3 (R2 = 0,75, p = 0,0001) et avec les niveaux de l'excrétion de sodium au jour 3 (R2 = 0,74, p = 0,0001). En outre, les inventeurs ont déterminé que la supplémentation en M101 a également une corrélation négative avec un résultat chronique (3 mois): avec la créatinine (R2 = 0,75, p = 0,0001), la protéinurie (R2 = 0,55, p = 0,013) et la fibrose (R2 = 0,78, p = 0,0001).

L'ANOVA a révélé qu'il y avait une interaction entre M101 et la solution utilisée pour un résultat aigu: avec la créatininémie (p = 0,001) et la réabsorption du sodium (p = 0,04) au jour 3 ; tandis que les effets chroniques de M101 après 3 mois sont indépendants de la solution utilisée (données non présentées).

### REFERENCES

[1] Salahudeen AK. Cold ischemic injury of transplanted kidneys: new insights from experimental studies. American journal of physiology. 2004 Aug;287(2):F181-7.
[2] Salahudeen AK. Cold ischemic injury of transplanted organs: some new stratégies against an old problem. Am J Transplant. 2004 Jan;4(1):1.
[3] Faure JP, Baumert H, Han Z, Goujon JM, Favreau F, Dutheil D, et al. Evidence for a protective role of trimetazidine during cold ischemia: targeting inflammation and nephron mass. Biochemical pharmacology. 2003 Dec 1;66(11):2241-50.
[4] Hauet T, Goujon JM, Vandewalle A, Baumert H, Lacoste L, Tillement JP, et al. Trimetazidine reduces rénal dysfunction by limiting the cold ischemia/reperfusion injury in autotransplanted pig kidneys. J Am Soc Nephrol. 2000 Jan; 11(1): 138-48.
[5] Jayle C, Favreau F, Zhang K, Doucet C, Goujon JM, Hebrard W, et al. Comparison of protective effects of trimetazidine against experimental warm ischemia of different durations: early and long-term effects in a pig kidney model. American journal of physiology. 2007 Mar;292(3):F1082-93.
[6] Simmons MN, Schreiber MJ, Gill IS. Surgical renal ischemia: a contemporary overview. The Journal of urology. 2008 Jul;180(1):19-30.
[7] Kosieradzki M, Rowinski W. Ischemia/reperfusion injury in kidney transplantation: mechanisms and prevention. Transplantation proceedings. 2008 Dec;40(10):3279-88.
[8] Favreau F, Thuillier R, Cau J, Milin S, Manguy E, Mauco G, et al. Anti-thrombin therapy during warm ischemia and cold preservation prevents chronic kidney graft fibrosis in a DCD model. Am J Transplant. Jan;10(1):30-9.
[9] Giraud S, Thuillier R, Belliard A, Hebrard W, Nadeau C, Milin S, et al. Direct thrombin inhibitor prevents delayed graft function in a porcine model of rénal transplantation. Transplantation. 2009 Jun 15;87(11):1636-44.
[10] Dragun D, Hoff U, Park JK, Qun Y, Schneider W, Luft FC, et al. Ischemia-reperfusion injury in renal transplantation is independent of the immunologic background. Kidney Int. 2000 Nov;58(5):2166-77.
[11] Koo DD, Welsh KI, Roake JA, Morris PJ, Fuggle SV. Ischemia/reperfusion injury in human kidney transplantation: an immunohistochemical analysis of changes after reperfusion. The American journal of pathology. 1998 Aug;153(2):557-66.
[12] Cassie S, Masterson MF, Polukoshko A, Viskovic MM, Tibbles LA. Ischemia/reperfusion induces the recruitment of leukocytes from whole blood under flow conditions. Free Radic Biol Med. 2004 May 1;36(9):1102-11.
[13] Belzer FO, Southard JH. Principles of solid-organ preservation by cold storage. Transplantation. 1988 Apr;45(4):673-6.
[14] Maathuis MH, Leuvenink HG, Ploeg RJ. Perspectives in organ preservation. Transplantation. 2007 May 27;83(10):1289-98.
[15] Minor T, Sitzia M, Dombrowski F. Kidney transplantation from non-heart-beating donors after oxygenated low-flow machine perfusion preservation with histidine-tryptophan-ketoglutarate solution. Transpl Int. 2005 Jan;17(11):707-12.
[16] Matsumoto S, Kuroda Y. Perfluorocarbon for organ preservation before transplantation. Transplantation. 2002 Dec 27;74(12):1804-9.
[17] Rousselot M, Delpy E, Drieu La Rochelle C, Lagente V, Pirow R, Rees JF, et al. Arenicola marina extracellular hemoglobin: a new promising blood substitute. Biotechnology journal. 2006 Mar;1(3):333-45.
[18] Rousselot M, Le Guen D, Zal F. Novel dissociation mechanism of a polychaetous annelid extracellular haemoglobin. FEBS J. 2006 Apr;273(7): 1582-96.
[19] Zal F, Green BN, Lallier FH, Vinogradov SN, Toulmond A. Quaternary structure of the extracellular haemoglobin of the lugworm Arenicola marina: a multi-angle-laser-light-scattering and electrospray-ionisation-mass-spectrometry analysis. European journal of biochemistry / FEBS. 1997 Jan 15;243(1-2):85-92.
[20] Toulmond A. Blood oxygen transport and metabolism of the confined lugworm Arenicola marina (L.). The Journal of experimental biology. 1975 Dec;63(3):647-60.
[21] Toulmond A, Tchernigovtzeff C. Ventilation and respiratory gas exchanges of the lugworm Arenicola marina (L.) as functions of ambient PO2 (20-700 torr). Respiration physiology. 1984 Sep;57(3):349-63.
[22] Ahlenstiel T, Burkhardt G, Kohler H, Kuhlmann MK. Improved cold preservation of kidney tubular cells by means of adding bioflavonoids to organ preservation solutions. Transplantation. 2006 Jan 27;81(2):231-9.
[23] McCord JM. Oxygen-derived free radicals in postischemic tissue injury. The New England journal of medicine. 1985 Jan 17;312(3):159-63.
[24] Chabasse C, Bailly X, Rousselot M, Zal F. The multigenic family of the extracellular hemoglobin from the annelid polychaete Arenicola marina. Comp Biochem Physiol B Biochem Mol Biol. 2006 Jul;144(3):319-25.
[25] Royer WE, Jr., Omartian MN, Knapp JE. Low resolution crystal structure of Arenicola erythrocruorin: influence of coiled coils on the architecture of a megadalton respiratory protein. J Mol Biol. 2007 Jan 5;365(1):226-36.
[26] Favreau F, Thuillier R, Cau J, Milin S, Manguy E, Mauco G, et al. Anti-thrombin Therapy During Warm Ischemia and Cold Preservation Prevents Chronic Kidney Graft Fibrosis in a DCD Model. Am J Transplant. 2009 Dec 2.
[27] Bernhardt WM, Gottmann U, Doyon F, Buchholz B, Campean V, Schodel J, et al. Donor treatment with a PHD-inhibitor activating HIFs prevents graft injury and prolongs survival in an allogenic kidney transplant model. Proceedings of the National Academy of Sciences of the United States of America. 2009 Nov 23.
[28] Bos EM, Leuvenink HG, Snijder PM, Kloosterhuis NJ, Hillebrands JL, Leemans JC, et al. Hydrogen Sulfide-Induced Hypometabolism Prevents Renal Ischemia/Reperfusion Injury. J Am Soc Nephrol. 2009 Jul 23.
[29] Hosgood SA, Nicholson ML. Hydrogen sulphide ameliorates ischaemia-reperfusion injury in an experimental model of non-heart-beating donor kidney transplantation. The British journal of surgery. 2009 Dec 23.
[30] Kumar S, Allen DA, Kieswich JE, Patel NS, Harwood S, Mazzon E, et al. Dexamethasone Ameliorates Renal Ischemia-Reperfusion Injury. J Am Soc Nephrol. 2009 Sep 24.
[31] Yoshida J, Ozaki KS, Nalesnik MA, Ueki S, Castillo-Rama M, Faleo G, et al. Ex vivo Application of Carbon Monoxide in UW Solution Prevents Transplant-Induced Renal Ischemia/Reperfusion Injury in Pigs. Am J Transplant. Feb 25.
[32] Companiesandmarkets.com. Organ Preservation Solutions - A Global Strategic Business Report. 2010 [cited; Available from: http://www.companiesandmarkets.com/Summary-Market-Report/organ-preservation-solutions-a-global-strategic-business-report-317184.asp
[33] Pereira-Sampaio MA, Favorito LA, Sampaio FJ. Pig kidney: anatomical relationships between the intrarenal arteries and the kidney collecting system. Applied study for urological research and surgical training. The Journal of urology. 2004 Nov;172(5 Pt 1):2077-81.
[34] Giraud S, Favreau F, Chatauret N, Thuillier R, Maiga S, Hauet T. Contribution of Large Pig for Renal Ischemia-Reperfusion and Transplantation Studies: The Preclinical Model. Journal of Biomedicine and Biotechnology. in press.
[35] Assendelft, ed. Spectrophotometry of haemoglobin derivatives. Assen, The netherlands: Charles C Thomas Publisher, Royal Vangorcum LTD 1970.
[36] Oberley LW, Spitz DR. Assay of superoxide dismutase activity in tumor tissue. Methods Enzymol. 1984;105:457-64.
[37] Dutheil D, Rioja-Pastor I, Tallineau C, Goujon JM, Hauet T, Mauco G, et al. Protective effect of PEG 35,000 Da on renal cells: paradoxical activation of JNK signaling pathway during cold storage. Am J Transplant. 2006 Jul;6(7):1529-40.
[38] Hauet T, Goujon JM, Baumert H, Petit I, Carretier M, Eugene M, et al. Polyethylene glycol reduces the inflammatory injury due to cold ischemia/reperfusion in autotransplanted pig kidneys. Kidney international. 2002 Aug;62(2):654-67.
[39] Solez K, Colvin RB, Racusen LC, Haas M, Sis B, Mengel M, et al. Banff 07 classification of renal allograft pathology: updates and future directions. Am J Transplant. 2008 Apr;8(4):753-60.
[40] Grimm PC, Nickerson P, Gough J, McKenna R, Stern E, Jeffery J, et al. Computerized image analysis of Sirius Red-stained renal allograft biopsies as a surrogate marker to predict long-term allograft function. J Am Soc Nephrol. 2003 Jun;14(6):1662-8.
[41] Favreau F, Rossard L, Zhang K, Desurmont T, Manguy E, Belliard A, et al. Expression and modulation of translocator protein and its partners by hypoxia reoxygenation or ischemia and reperfusion in porcine renal models. American journal of physiology. 2009 Jul;297(1):F177-90.
[42] Thuillier R, Favreau F, Celhay O, Macchi L, Milin S, Hauet T. Thrombin inhibition during kidney ischemia-reperfusion reduces chronic graft inflammation and tubular atrophy. Transplantation. 2010 Sep 27;90(6):612-21.
[43] Fotakis G, Timbrell JA. In vitro cytotoxicity assays: comparison of LDH, neutral red, MTT and protein assay in hepatoma cell lines following exposure to cadmium chloride. Toxicol Lett. 2006 Jan 5;160(2):171-7.
[44] Gallucci S, Matzinger P. Danger signals: SOS to the immune system. Current opinion in immunology. 2001 Feb;13(1):114-9.
[45] El-Zoghby ZM, Stegall MD, Lager DJ, Kremers WK, Amer H, Gloor JM, et al. Identifying specific causes of kidney allograft loss. Am J Transplant. 2009 Mar;9(3):527-35.
[46] Copeland JW, Beaumont BW, Merrilees MJ, Pilmore HL. Epithelial-to-mesenchymal transition of human proximal tubular epithelial cells: effects of rapamycin, mycophenolate, cyclosporin, azathioprine, and methylprednisolone. Transplantation. 2007 Mar 27;83(6):809-14.
[47] Nankivell BJ, Chapman JR. Chronic allograft nephropathy: current concepts and future directions. Transplantation. 2006 Mar 15;81(5):643-54.
[48] t Hart NA, van der Plaats A, Faber A, Leuvenink HG, Olinga P, Wiersema-Buist J, et al. Oxygenation during hypothermic rat liver preservation: an in vitro slice study to demonstrate beneficial or toxic oxygenation effects. Liver Transpl. 2005 Nov;11(11):1403-11.
[49] Kuroda Y, Kawamura T, Suzuki Y, Fujiwara H, Yamamoto K, Saitoh Y. A new, simple method for cold storage of the pancreas using perfluorochemical. Transplantation. 1988 Sep;46(3):457-60.
[50] Fujino Y, Kuroda Y, Suzuki Y, Fujiwara H, Kawamura T, Morita A, et al. Preservation of canine pancreas for 96 hours by a modified two-layer (UW solution/perfluorochemical) cold storage method. Transplantation. 1991 May;51(5):1133-5.
[51] Yoshikawa T, Suzuki Y, Fujino Y, Kakinoki K, Li S, Goto T, et al. Detailed analysis of mucosal restoration of the small intestine after the cavitary two-layer cold storage method. Am J Transplant. 2005 Sep;5(9):2135-42.
[52] Farrar D, Grocott M. Intravenous artificial oxygen carriers. Hosp Med. 2003 Jun;64(6):352-6.
[53] Cataldi A. Cell responses to oxidative stressors. Current pharmaceutical design.16(12):1387-95.
[54] Hosgood SA, Mohamed IH, Nicholson ML. The two layer method does not improve the preservation of porcine kidneys. Med Sci Monit.17(1):BR27-33.
[55] Rolles K, Foreman J, Pegg DE. A pilot clinical study of retrograde oxygen persufflation in renal preservation. Transplantation. 1989 Aug;48(2):339-42.
[56] Kakehata J, Yamaguchi T, Togashi H, Sakuma I, Otani H, Morimoto Y, et al. Therapeutic Potentials of an Artificial Oxygen-Carrier, Liposome-Encapsulated Hemoglobin, for Ischemia/Reperfusion-Induced Cerebral Dysfunction in Rats. Journal of pharmacological sciences. Sep 11.
[57] Spahn DR, Kocian R. Artificial 02 carriers: status in 2005. Current pharmaceutical design. 2005;11(31):4099-114.
[58] Regner KR, Nilakantan V, Ryan RP, Mortensen J, White SM, Shames BD, et al. Protective effect of Lifor solution in experimental renal ischemia-reperfusion injury. The Journal of surgical research. 2010 Dec;164(2):e291-7.
[59] Jouan L, Taveau JC, Marco S, Lallier FH, Lamy JN. Occurrence of two architectural types of hexagonal bilayer hemoglobin in annelids: comparison of 3D reconstruction volumes of Arenicola marina and Lumbricus terrestris hemoglobins. J Mol Biol. 2001 Jan 26;305(4):757-71.
[60] Weber RE, Vinogradov SN. Nonvertebrate hemoglobins: functions and molecular adaptations. Physiol Rev. 2001 Apr;81(2):569-628.
[61] Patel S, Spencer CP. Studies on the Haemoglobin of Arenicola Marina. Comp Biochem Physiol. 1963 Feb;16:65-82.

## Revendications

1. Utilisation d'une composition comprenant au moins une globine d'hémoglobine extracellulaire *d'Arenicolidae,* un protomère de globine d'hémoglobine extracellulaire *d'Arenicolidae* ou une hémoglobine extracellulaire *d'Arenicolidae ,* une solution de stabilisation et/ou une solution de conservation d'organes, ladite composition ayant une température comprise entre 0°C et 37°C, pour préserver un organe chez un donneur décédé en état de mort cérébrale ou décédé d'arrêt cardiaque.

2. Utilisation selon la revendication 1, **caractérisée en ce que** l'hémoglobine extracellulaire *d'Arenicolidae* est l'hémoglobine extracellulaire *d'Arenicola marina.*

3. Utilisation selon l'une des revendications 1 à 2, **caractérisée en ce que** la solution de stabilisation est une solution aqueuse comprenant des sels, et confère à la composition selon l'invention un pH compris entre 6.5 et 7.6.

4. Utilisation selon la revendication 3, **caractérisée en ce que** la solution de stabilisation est une solution aqueuse comprenant des ions chlorure, sodium, calcium, magnésium et potassium.

5. Utilisation selon l'une des revendications 1 à 4, **caractérisée en ce que** la solution de stabilisation est une solution aqueuse comprenant 90 mM de NaCl, 23 Mm de Na-gluconate, 2,5 mM de CaCl₂, 27 mM de Na-acétate, 1,5 mM de MgCl₂, 5 mM de KCI, et a un pH de 7,1 ± 0,5.

6. Utilisation selon l'une des revendications 1 à 5, **caractérisée en ce que** la solution de conservation d'organe est une solution aqueuse ayant un pH compris entre 6.5 et 7.5 et comprenant des sels ; des sucres; des antioxydants ; des agents actifs; et éventuellement des colloïdes.

7. Utilisation selon la revendication 6, **caractérisée en ce que** la solution de conservation d'organe est une solution aqueuse ayant un pH compris entre 6.5 et 7.5 et comprenant :
- des sels choisis parmi des ions chlorure, sulfate, sodium, calcium, magnésium et potassium ;
- des sucres choisis parmi le mannitol, le raffinose, le saccharose, le glucose, le fructose, le lactobionate et le gluconate ;
- du glutathion ;
- des agents actifs choisis parmi des inhibiteurs de xanthine oxydase tel que l'allopurinol, des lactates et des acides aminés, tel que l'histidine, l'acide glutamique, le tryptophane ;
- et éventuellement des colloïdes choisis parmi l'hydroxyéthyl amidon, le polyéthylène glycol et le dextran.

8. Utilisation selon l'une des revendications 1 à 7, **caractérisée en ce que** l'hémoglobine extracellulaire *d'Arenicolidae,* ses protomères de globine et/ou ses globines, est présente à une concentration, par rapport au volume final de composition, comprise entre 0,001 mg/ml et 100 mg/ml, et **en ce que** la composition a une osmolarité comprise entre 250 et 350 mOsm/L.

9. Utilisation selon la revendication 8, **caractérisée en ce que** l'hémoglobine extracellulaire *d'Arenicolidae,* ses protomères de globine et/ou ses globines, est présente à une concentration, par rapport au volume final de composition, comprise entre 1 mg/ml et 5 mg/ml, et **en ce que** la composition a une osmolarité comprise entre 275 et 310 mOsm/L, de préférence d'environ 302 mOsm/L.

10. Procédé de conservation d'un organe ex situ dans un donneur décédé en état de mort cérébrale ou décédé d'arrêt cardiaque, comprenant les étapes suivantes:
a) perfusion dudit donneur décédé avec une composition selon l'une des revendications 1 à 9; puis
b) prélèvement de l'organe à transplanter ; puis
c) conservation en statique ou en perfusion dynamique dudit organe obtenu en b), à une température comprise entre 0°C et 37°C, préférentiellement comprise entre 2°C et 25°C, plus préférentiellement d'environ 4°C, pendant un temps déterminé en fonction dudit organe, dans la composition ou la solution aqueuse définie à l'étape a).

11. Procédé de conservation d'un organe ex situ selon la revendication 10, **caractérisé en ce que** l'étape c) de conservation en statique ou en perfusion dynamique dudit organe obtenu en b) se fait à une température comprise entre 2°C et 25°C.

12. Composition ayant un pH compris entre 6.5 et 7,6, qui comprend :
- au moins une globine, un protomère de globine ou une hémoglobine extracellulaire d'*Arenicolidae,*
- des ions calcium;
- du KOH;
- du NaOH;
- du KH₂PO₄;
- du MgCl₂;
- au moins un sucre choisi parmi le raffinose et le glucose;
- de l'adénosine;
- du glutathion;
- de l'allopurinol; et
- au moins un composé choisi parmi l'hydroxyéthyl amidon, les polyéthylène glycols de différents poids moléculaires et la sérum albumine humaine.

13. Composition selon la revendication 12, qui comprend :
- au moins une globine, un protomère de globine ou une hémoglobine extracellulaire d'*Arenicolidae,*
- des ions calcium en quantité comprise entre 0 et 0.5 mM ;
- du KOH en quantité comprise entre 20 et 100 mM ;
- du NaOH en quantité comprise entre 20 et 125 mM ;
- du KH₂PO₄ en quantité comprise entre 20 et 25 mM ;
- du MgCl₂ en quantité comprise entre 3 et 5 mM ;
- au moins un sucre choisi parmi le raffinose et le glucose en quantité comprise entre 5 et 200 mM;
- de l'adénosine en quantité comprise entre 3 et 5 mM ;
- du glutathion en quantité comprise entre 2 et 4 mM ;
- de l'allopurinol en quantité comprise entre 0 et 1 mM ; et
- au moins un composé choisi parmi l'hydroxyéthyl amidon, les polyéthylène glycols de différents poids moléculaires et la sérum albumine humaine, en quantité comprise entre 1 et 50 g/L.

## Patentansprüche

1. Verwendung einer Zusammensetzung, umfassend mindestens ein Globin eines extrazellulären Hämoglobins von *Arenicolidae,* ein Protomer von Globin von extrazellulärem Hämoglobin von *Arenicolidae* oder ein extrazelluläres Hämoglobin von *Arenicolidae,* eine Stabilisierungslösung und/oder eine Konservierungslösung für Organe, wobei die Zusammensetzung eine Temperatur zwischen 0 °C und 37 °C aufweist, zum Erhalten eines Organs bei einem verstorbenen Spender nach Eintreten des Hirntodes oder Herzversagen.

2. Verwendung nach Anspruch 1, **dadurch gekennzeichnet, dass** das extrazelluläre Hämoglobin von *Arenicolidae* extrazelluläres Hämoglobin von *Arenicola marina* ist.

3. Verwendung nach einem der Ansprüche 1 bis 2, **dadurch gekennzeichnet, dass** die Stabilisierungslösung eine wässrige Lösung ist, die Salze umfasst, und der erfindungsgemäßen Zusammensetzung einen pH-Wert zwischen 6,5 und 7,6 verleiht.

4. Verwendung nach Anspruch 3, **dadurch gekennzeichnet, dass** die Stabilisierungszusammensetzung eine wässrige Lösung ist, umfassend Chlorid-, Natrium-, Calcium-, Magnesium- und Kaliumionen.

5. Verwendung nach einem der Ansprüche 1 bis 4, **dadurch gekennzeichnet, dass** die Stabilisierungslösung eine wässrige Lösung ist, umfassend 90 mM NaCl, 23 mM Na-Gluconat, 2,5 mM CaCl₂, 27 mM Na-Acetat, 1,5 mM MgCl₂, 5 mM KCl und einen pH-Wert von 7,1 ± 0,5.

6. Verwendung nach einem der Ansprüche 1 bis 5, **dadurch gekennzeichnet, dass** die Lösung zur Organkonservierung eine wässrige Lösung ist, mit einem pH-Wert, umfassend zwischen 6,5 und 7,5, und umfassend Salze, Zucker, Antioxidationsmittel, Wirkstoffe und gegebenenfalls Kolloide.

7. Verwendung nach Anspruch 6, **dadurch gekennzeichnet, dass** die Lösung zur Organkonservierung eine wässrige Lösung ist, mit einem pH-Wert zwischen 6,5 und 7,5, und umfasst:
- Salze, ausgewählt aus Chlor-, Sulfat-, Natrium-, Calcium-, Magnesium- und Kaliumionen;
- Zucker, ausgewählt aus Mannitol, Raffinose, Saccharose, Glucose, Fructose, Lactobionat und Gluconat;
- Glutathion;
- Wirkstoffe, ausgewählt aus Inhibitoren von Xanthinoxidase, wie etwa Allopurinol, Lactaten und Aminosäuren, wie etwa Histidin, Glutaminsäure, Tryptophan;
- und gegebenenfalls Kolloide, ausgewählt aus Hydroxyethylstärke, Polyethylenglycol und Dextran.

8. Verwendung nach einem der Ansprüche 1 bis 7, **dadurch gekennzeichnet, dass** das extrazelluläre Hämoglobin von *Arenicoldae,* seine Promotomeren von Globin und/oder seinen Globinen bezüglich des Endvolumens der Zusammensetzung in einer Konzentration zwischen 0,001 mg/ml und 100 mg/ml vorliegen, und dadurch, dass die Zusammensetzung eine Osmolarität zwischen 250 und 350 mOsm/l aufweist.

9. Verwendung nach Anspruch 8, **dadurch gekennzeichnet, dass** das extrazelluläre Hämoglobin von *Arenicolidae,* seine Protomeren von Globin und/oder seine Globine in einer Konzentration bezüglich des Endvolumens der Zusammensetzung zwischen 1 mg/l und 5 mg/l vorliegen, und dadurch, dass die Zusammensetzung eine Osmolarität zwischen 275 und 310 mOsm/l, vorzugsweise etwa 302 mOsm/l, aufweist.

10. Verfahren zur ex situ-Konservierung eines Organs in einem verstorbenen Spender nach Eintreten des Hirntodes oder Herzversagen, umfassend die folgenden Schritte:
a) Infusion des verstorbenen Spenders mit einer Zusammensetzung nach einem der Ansprüche 1 bis 9; dann
b) Entnahme des Organs für eine Transplantation; dann
c) Konservierung durch statische oder durch dynamische Infusion des in b) erhaltenen Organs, bei einer Temperatur zwischen 0 °C und 37 °C, vorzugsweise zwischen 2 °C und 25 °C, mehr bevorzugt bei etwa 4 °C, für eine Zeitdauer, die bestimmt wird durch die Funktion des Organs, in der Zusammensetzung oder der wässrigen Lösung, wie in Schritt a) definiert.

11. Verfahren zur ex situ-Konservierung eines Organs nach Anspruch 10, **dadurch gekennzeichnet, dass** Schritt c) der Konservierung durch statische oder dynamische Infusion des in b) erhaltenen Organs bei einer Temperatur zwischen 2 °C und 25 °C erfolgt.

12. Zusammensetzung mit einem pH-Wert zwischen 6,5 und 7,6, umfassend:
- mindestens ein Globin, ein Protomer von Globin oder ein extrazelluläres Hämoglobin von *Arenicolidae,*
- Calciumionen;
- KOH;
- NaOH;
- KH₂PO₄;
- MgCl₂;
- mindestens einen Zucker, ausgewählt aus Raffinose und Glucose;
- Adenosin;
- Glutathion;
- Allopurinol; und
- mindestens eine Zusammensetzung, ausgewählt aus Hydroxyethylstärke, Polyethylenglykolen, mit verschieden Molekulargewichten und humanem Serumalbumin.

13. Zusammensetzung nach Anspruch 12, umfassend:
- mindestens ein Globin, ein Protomer von Globin oder ein extrazelluläres Hämoglobin von *Arenicolidae,*
- Calciumionen, in einer Menge zwischen 0 und 0,5 nM;
- KOH, in einer Menge zwischen 20 und 100 mM;
- NaOH, in einer Menge zwischen 20 und 125 mM ;
- KH₂PO₄, in einer Menge zwischen 20 und 25 mM;
- MgCl₂, in einer Menge zwischen 3 und 5 mM;
- mindestens einen Zucker, ausgewählt aus Raffinose und Glucose, in einer Menge zwischen 5 und 200 mM;
- Adenosin, in einer Menge zwischen 3 und 5 mM;
- Glutathion, in einer Menge zwischen 2 und 4 mM;
- Allopurinol, in einer Menge zwischen 0 und 1 mM; und
- mindestens eine Zusammensetzung, ausgewählt aus Hydroxyethylstärke, Polyethylenglykolen mit verschieden Molekulargewichten und humanem Serumalbumin, in einer Menge zwischen 1 und 50 g/l.

## Claims

1. Use of a composition comprising at least one globin of extracellular haemoglobin from *Arenicolidae,* one globin protomer of extracellular haemoglobin from *Arenicolidae* or one extracellular haemoglobin from *Arenicolidae,* a stabilizing solution and/or an organ-preserving solution, said composition having a temperature of between 0°C and 37°C, for preserving an organ in a donation after brain death donor or a donation after cardiac death donor.

2. Use according to Claim 1, **characterized in that** the extracellular haemoglobin of *Arenicolidae* is the extracellular haemoglobin of *Arenicola marina.*

3. Use according to either of Claims 1 and 2, **characterized in that** the stabilizing solution is an aqueous solution comprising salts, and confers on the composition according to the invention a pH of between 6.5 and 7.6.

4. Use according to Claim 3, **characterized in that** the stabilizing solution is an aqueous solution comprising chloride, sodium, calcium, magnesium and potassium ions.

5. Use according to one of Claims 1 to 4, **characterized in that** the stabilizing solution is an aqueous solution comprising 90 mM of NaCl, 23 mM of Na gluconate, 2.5 mM of CaCl₂, 27 mM of Na acetate, 1.5 mM of MgCl₂, 5 mM of KC1, and a pH of 7.1 ± 0.5.

6. Use according to one of Claims 1 to 5, **characterized in that** the organ-preserving solution is an aqueous solution having a pH of between 6.5 and 7.5 and comprising salts; sugars; antioxidants; active agents; and optionally colloids.

7. Use according to Claim 6, **characterized in that** the organ-preserving solution is an aqueous solution having a pH of between 6.5 and 7.5 and comprising:
- salts chosen from chloride, sulphate, sodium, calcium, magnesium and potassium ions;
- sugars chosen from mannitol, raffinose, sucrose, glucose, fructose, lactobionate and gluconate;
- glutathione;
- active agents chosen from xanthine oxidase inhibitors such as allopurinol, lactates and amino acids, such as histidine, glutamic acid or tryptophan;
- and optionally colloids chosen from hydroxyethyl starch, polyethylene glycol and dextran.

8. Use according to one of Claims 1 to 7, **characterized in that** the extracellular haemoglobin from *Arenicolidae,* globin protomers thereof and/or globins thereof is (are) present at a concentration, relative to the final volume of composition, of between 0.001 mg/ml and 100 mg/ml, and **in that** the composition has an osmolarity of between 250 and 350 mOsm/l.

9. Use according to Claim 8, **characterized in that** the extracellular haemoglobin from *Arenicolidae,* globin protomers thereof and/or globins thereof is (are) present at a concentration, relative to the final volume of composition, of between 1 mg/ml and 5 mg/ml and **in that** the composition has an osmolarity of between 275 and 310 mOsm/l, preferably of approximately 302 mOsm/l.

10. Method for preserving an organ ex situ in a donation after brain death donor or a donation after cardiac death donor, comprising the following steps:
a) perfusion of said deceased donor with a composition according to one of Claims 1 to 9; then
b) harvesting of the organ to be transplanted; then
c) static or dynamic-perfusion preservation of said organ obtained in b), at a temperature of between 0°C and 37°C, preferentially of between 2°C and 25°C, more preferentially of approximately 4°C, for a time predetermined according to said organ, in the composition or the aqueous solution defined in step a).

11. Method for preserving an organ ex situ according to Claim 10, **characterized in that** step c) of static or dynamic-perfusion preservation of said organ obtained in b) is carried out at a temperature of between 2°C and 25°C.

12. Composition having a pH of between 6.5 and 7.6, which comprises:
- at least one globin, one globin protomer or one extracellular haemoglobin from *Arenicolidae;*
- calcium ions;
- KOH;
- NaOH;
- KH₂PO₄;
- MgCl₂;
- at least one sugar chosen from raffinose and glucose;
- adenosine;
- glutathione;
- allopurinol; and
- at least one compound chosen from hydroxyethyl starch, polyethylene glycols of various molecular weights and human serum albumin.

13. Composition according to Claim 12, which comprises:
- at least one globin, one globin protomer or one extracellular haemoglobin from *Arenicolidae;*
- calcium ions in an amount of between 0 and 0.5 mM;
- KOH in an amount of between 20 and 100 mM;
- NaOH in an amount of between 20 and 125 mM;
- KH₂PO₄ in an amount of between 20 and 25 mM;
- MgCl₂ in an amount of between 3 and 5 mM;
- at least one sugar chosen from raffinose and glucose in an amount of between 5 and 200 mM;
- adenosine in an amount of between 3 and 5 mM;
- glutathione in an amount of between 2 and 4 mM;
- allopurinol in an amount of between 0 and 1 mM; and
- at least one compound chosen from hydroxyethyl starch, polyethylene glycols of various molecular weights and human serum albumin, in an amount of between 1 and 50 g/l.
